(19) Europäisches Patentamt
European Patent Office
Office européen des brevets

(11) EP 1 272 673 B1

(12) **EUROPEAN PATENT SPECIFICATION**

(45) Date of publication and mention of the grant of the patent:
**14.03.2007 Bulletin 2007/11**

(21) Application number: **01929454.5**

(22) Date of filing: **26.03.2001**

(51) Int Cl.:
***C12Q 1/68*** *(2006.01)*

(86) International application number:
**PCT/EP2001/003397**

(87) International publication number:
**WO 2001/071028 (27.09.2001 Gazette 2001/39)**

(54) **SPECIFIC MULTIPLEX ANALYSIS OF NUCLEIC ACIDS**

SPEZIFISCHE MULTIPLEX-ANALYSE VON NUKLEINSÄUREN

ANALYSE MULTIPLEX SPECIFIQUE D'ACIDES NUCLEIQUES

(84) Designated Contracting States:
**AT BE CH CY DE DK ES FI FR GB GR IE IT LI LU MC NL PT SE TR**

(30) Priority: **24.03.2000 US 191760 P**

(43) Date of publication of application:
**08.01.2003 Bulletin 2003/02**

(73) Proprietor: **Olympus Corporation**
**Shibuya-ku, Tokyo (JP)**

(72) Inventors:
- **REUBER, Dr. Bernadette**
  **D-73553 Alfdorf (DE)**
- **WEBER, Dr.Stephanie (neé MUCK)**
  **D-42653 Solingen (DE)**
- **WEINER, Dr. Olaf**
  **D-90429 Nürnberg (DE)**
- **ZIRWES, Rudolf**
  **41352 Korschenbroich (DE)**

(74) Representative: **Meyers, Hans-Wilhelm et al**
**Patentanwälte**
**von Kreisler-Selting-Werner**
**Postfach 10 22 41**
**50462 Köln (DE)**

(56) References cited:
**WO-A-96/40995**
**WO-A-97/42345**
**WO-A-98/24928**

- **BOLDT B. ET AL.,: "one-tube method for complete HPA-1 genotyping by PCR using sequence-specific primers" BR. J. HAEMATOLOGY, vol. 99, December 1997 (1997-12), pages 968-973, XP002210179**
- **ECHEVARRIA J E ET AL: "SIMULTANEOUS DETECTION AND IDENTIFICATION OF HUMAN PARAINFLUENZA VIRUSES 1, 2, AND 3 FROM CLINICAL SAMPLES BY MULTIPLEX PCR" JOURNAL OF CLINICAL MICROBIOLOGY, WASHINGTON, DC, US, vol. 36, no. 5, May 1998 (1998-05), pages 1388-1391, XP000876835 ISSN: 0095-1137**
- **DAHLEN P. E AL.,: "the use of europium (Eu3+) labelled primers in PCR amplification of specific target DNA" MOL. CELL. PROBES, vol. 5, no. 2, - April 1991 (1991-04) pages 143-149, XP002210180**
- **SHU YE ET AL: "ALLELE SPECIFIC AMPLIFICATION BY TETRA-PRIMER PCR" NUCLEIC ACIDS RESEARCH, OXFORD UNIVERSITY PRESS, SURREY, GB, vol. 20, no. 5, 11 March 1992 (1992-03-11), page 1152 XP000371878 ISSN: 0305-1048**

EP 1 272 673 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention provides a novel type of amplification and detection method that allows homogeneous multiplex analysis of nucleic acid sequences and the detection of single nucleotide polymorphisms and nucleotide sequence changes such as point-mutations, variant alleles, deletions, insertions, repeats and/or inversions. Additionally, the invention may be useful for the quantification of nucleic acids and genotyping.

Homogeneous assays for the detection of nucleic acid sequences, especially for the detection of single-nucleotide polymorphisms (SNPs), are a critical tool for genetic analysis and have been developed into commercial products particularly for clinical diagnostics. SNPs are evolutionary stable point mutations scattered throughout the human genome at every 500 to 1000 DNA bases and, therefore, represent the most common genetic variation in human populations (Cooper et al., *Hum Genet* 1985, 69:201-205; Collins et al., *Genome Res* 1998, 8:1229-1231). The genome of any two non-related individuals is estimated to differ in at least three million nucleotide positions, approximately 500,000 located in coding regions. The availability of the complete sequence of the human genome (International Human Genome Sequencing Consortium, *Nature* 2001, 409:860-921) now provides the basis for the generation of a whole-genome SNP map, a powerful tool for genetic analysis which will uncover the association of SNPs with many disease traits, and holds great promise for the optimization of new drug development and the individualization of clinical diagnostics and therapeutics (Bentley, *Med Res Rev* 2000, 20:189-196; Pfost et al., *Trends Biotechnol* 2000, 18:334-338; Schork et al., *Clin Genet* 2000, 58:250-264). Future developments will focus on identifying and/or screening few dozens to hundreds of SNPs relevant to certain disease areas (e.g. cancer, neurodegenerative or cardiovascular disease) rather than performing whole-genome SNP scans for each individual. Methods meeting the requirements of these developments must allow flexible and rapid assays, medium- to high-throughput and high-quality genotyping for SNPs of interest. Currently applied methods for the analysis of nucleic acid sequences, especially the analysis of SNPs, include restriction fragment-length polymorphism analysis of polymerase chain reaction products (RFLP-PCR), hybridization with allele-specific oligonucleotides (Nickerson et al., *Proc Natl Acad Sci USA* 1990, 87:8923-8927; Saiki et al., *Proc Natl Acad Sci USA* 1989, 86:6230-6234; de Verlaan et al., *Gene* 1986, 50:313-320; Wallace et al., *Nucleic Acids Res* 1981, 9:879-894; Zhang et al., *Nucleic Acids Res* 1991, 19:3929-3933), allele-specific PCR (Gibbs et al., *Nucleic Acids Res* 1989, 17:2437-2448; Newton et al., *Nucleic Acids Res* 1989, 17:2503-2516), oligonucleotide ligation assay (Grossman et al., *Nucleic Acids Res* 1994, 22:4527-4534; Landegren et al., *Science* 1988, 241:1077-1080), allele-specific ligase chain reaction (Abravaya et al., *Nucleic Acids Res* 1995, 23:675-682; Barany, *Proc Natl Acad Sci USA* 1991, 88:189-193; Wu und Wallace, *Genomics* 1989,4:560-569), high-density chip arrays for allele-specific hybridization analysis (Wang et al., *Science* 1998, 280:1077-1082; Yershov et al., *Proc Natl Acad Sci USA* 1996, 93:4913-4918), the 5'-nuclease assay (Holland et al., *Proc Natl Acad Sci USA* 1991, 88:7276-7280; Lee et al., *Biotechniques* 1999, 27:342-349; Livak, *Genet Anal* 1999, 14:143-149; Livak et al., *PCR Methods Appl* 1995, 4:357-362), the template-directed dye-terminator incorporation assay (Chen and Kwok, *Nucleic Acids Res* 1997, 25:347-353; Chen et al., *Genome Res* 1999, 9:492-498 and *Proc Natl. Acad Sci USA* 1997, 94:10756-10761), the molecular beacon allele-specific oligonucleotide assay (Marras et al., *Genet Anal* 1999, 14:151-156; Tyagi and Kramer, *Nat Biotechnol* 1996, 14:303-308), the allele-specific fluorescence energy transfer (LightCycler) assay (Bernard et al., *Anal Biochem* 1998, 255:101-107; Lay and Wittwer, *Clin Chem* 1997, 43:2262-2267), the structure-specific invasive endonuclease cleavage assay (Lyamichev et al., *Nat Biotechnol* 1999, 17:292-296), and the pyrosequencing assay (Ahmadian et al., *Anal Biochem* 2000, 280:103-110, Alderbom et al., *Genome Res* 2000, 10:1249-1258).

Most of the methods currently employ macroscopic fluorescence techniques, encompassing conventional fluorescence intensity, fluorescence polarization, energy transfer, or fluorescence quenching, which monitor the average fluorescence output from the ensemble of emitting fluorophores. In contrast, the single-molecule detection technology fluorescence correlation spectroscopy (FCS) (Eigen and Rigler, *Proc Natl Acad Sci USA* 1994, 91:5740-5747; Elson and Magde, *Biopolymers* 1974, 13:1-27, Magde et al., *Phys Rev Lett* 1972, 29:705-708 and *Biopolymers* 1974, 13: 29-61) statistically samples the fluorescence fluctuations of single molecules in discrete time intervals with a temporal resolution down to nanoseconds and below. Many such detection events are then averaged to define the properties of the whole assay system. Analysis of single molecular events is accomplished using confocal optics with an illumination/observation volume of approximately 0.24 fl ($10^{-15}$ liter) and has several advantages over conventional macroscopic fluorescence methods. For example, problems derived from probe adsorption, autofluorescence of reaction components or of reaction vessels, as well as inner filter effects are virtually eliminated. This results in a high signal-to-noise ratio and maximum sensitivity which is essentially independent of the assay volume and allows ultimate assay miniaturization down to 1 ml or below, an essential prerequisite of high-throughput analysis. Autocorrelation of fluorescence fluctuations provides multiple fluoresence parameters including the average numbers and translational diffusion times of fluorescent molecules in the confocal illumination/observation volume. FCS has been successfully applied to monitor molecular interactions as diverse as drug/target-interactions (Auer et al., *Drug Discovery Today* 1998, 3:457-465; Sterrer and Henco, *J Recept Signal Transduct Res* 1997, 17:511-520), enzymatic and binding studies (Kettling et al., *Proc Natl Acad Sci USA* 1998, 95:1416-1420; Meyer-Almes and Auer, *Biochemistry* 2000, 39:13261-13268) as well as protein aggregation studies (Pitschke et al., *Nat Med* 1998, 4:832-834, Tjemberg et al., *Chem Biol* 1999, 6:53-62). In addition, FCS has been shown to be well-suited for qualitative and quantitative nucleic acid analysis (Bjorling et al., *Biochemistry* 1998, 37:12971-12978; Kinjo, *BioTechniques* 1998, 25:705-6; Kinjo et al., *Anal Biochem* 1998, 260:166-172; Kinjo and Rigler, *Nucleic Acids Res* 1995, 23:1795-1799; Rigler et al., *J Biotechnol* 1998, 63:97-109; Walter et al., *Proc Natl Acad Sci USA* 1996, 93:12805-12810; Weiner et al., *Digestion* 2000, 61:84-89) and for indirect detection of point mutations in genes (Kinjo and Nishimura, *Bioimaging* 1997, 5:134-138).

European Patent No. 332435 ("Method of detecting nucleotide sequences") of Zeneca Ltd., London, and Newton et al. (*Nucleic Acid Res* 1989, 17:2503-16) describe a method for detecting the presence or absence of at least one nucleotide sequence variation. This method is known as ARMS (amplification refractory mutation system). The basis of the invention as described in European Patent No. 332435 is that oligonucleotides with a mismatched 3'-residue will not function as primers in a PCR reaction under appropriate conditions. The method comprises (i) contacting a nucleic acid sample with a diagnostic primer which is substantially complementary to a diagnostic portion of a target nucleic acid sequence, whereby extension of the diagnostic primer on a target template under appropriate conditions is only achieved where a terminal nucleotide of the diagnostic primer is complementary to either a suspected variant nucleotide or a corresponding normal nucleotide of the target nucleic acid sequence, and (ii) detecting the presence or absence of an extension product. According to the invention described in European Patent No. 332435, a preferred method of detecting and distinguishing between multiple amplification products generated by the method comprises selecting the position of the amplification primers on the target nucleic acid sequence such that the length of each amplification product is different. Accordingly, this may be accomplished by varying the distance of the amplification primers from the position of the labeling primers such that each variant nucleotide is associated with an amplification product of different length. The amplification products of different length may then be detected by established electrophoretic techniques. The above described detection technique, however, has the disadvantage of being time-consuming and non-homogeneous, entailing several manual worksteps. Additionally, the technique may only be used for simultaneous screening of a sample of target nucleic acid sequences for either the presence or absence of variant nucleotides in multiple but different positions on said target nucleic acid sequences, whereas a simultaneous analysis of multiple variant nucleotides situated in a defined single position is not feasible. Furthermore, the invention described in European Patent No. 332435 does not allow for the quantification of target nucleic acid sequences.

The international application WO 98/24928 discloses a method for detection of the presence or absence of chromosomal abnormalities which are associated with a condition in a subject and are each defined by at least one characteristic nucleic acid sequence. The method comprises subjecting a sample of nucleic acids to a multiplex molecular amplification procedure which comprises the use of at least 7 mutually distinct primers in one single reaction mixture, each of the primers defining an end of at least one characteristic nucleic acid sequence. At least one of the primers defines the first ends of at least two characteristic nucleic acid sequences, said two sequences being defined in their opposite ends by mutually distinct primers selected from the remainder of the primers, whereby the number of amplified characteristic nucleic acid sequences which can be detected upon conclusion of the amplification reaction is at least $1/2 \times n+1$.

The publication by Boldt B. et al. "One-tube method for complete HPA-1 genotyping by PCR using sequence-specific primers", *British Journal of Haematology,* 1997, 99, 968-973 describes a method to simultaneously and completely genotype both alleles of interest in a single PCR using a recombinant template that can only be amplified by the sequence-specific primers.

In the article by Echevarria J. et al. "Simultaneous detection and identification of human parainfluenza viruses 1, 2 and 3 from clinical samples by multiplex PCR", *Journal of Clinical Microbiology,* May 1998, p. 1388-1391 multiplex RT-PCR assays are disclosed using a mixture of three pairs of primers for primary amplification. A second amplification was performed for typing with a mixture of nested primers, which were optionally coupled with a fluorescent agent.

The WO 96/40995 discloses methods for detecting and quantitating gene sequences, such as mutated genes and oncogenes, in biological fluids. The fluid sample is obtained, deproteinized and the DNA present in the sample is extracted. The DNA is then amplified using an amplification procedure, such as PCR or LCR, to amplify the mutated gene sequence. In one embodiment, the DNA is contacted with a peptide nucleic acid prior to or during the amplification procedure.

The WO 97/42345 describes a method for the detection of diagnostic base sequences in a sample nucleic acid. Diagnostic primers having a non-complementary tail comprising tag and detector regions for use in the method. The method is of use in combination with the Amplification Refractory Mutation System (ARMS) for the detection of variant diagnostic base sequences against a background of normal diagnostic base sequences.

The present invention solves the above described limitations and features a homogeneous, sensitive, simple, and labor-saving technology for the multiplex analysis of nucleic acid sequences termed GALIOS (gene amplification and labeling in one system). The novel method is particularly useful for the direct detection of known SNPs, point-mutations, variant alleles, deletions, insertions, repeats and/or inversions. The current invention features a method which utilizes the high specificities of semi-nested polymerase chain reaction (Haff, *PCR Methods Appl* 1994, 3:332-337) with an improved method of variant nucleotide-specific amplification. The improved variant nucleotide-specific amplification method is characterized by the use of at least two substantially similar but distinguishable labeling primers for the analysis of a target nucleic acid sequence. The inventive method offers the advantage over other systems in that it enables amplification, labeling, and monitoring in a homogeneous format. According to the present invention, multiple reaction products can be monitored, analyzed and quantified directly and simultaneously in a mix-and-measure format, without any post-amplification processing or physical separation steps. The monitoring is preferably accomplished by the technique of fluorescence correlation spectroscopy (FCS).

According to one feature of the present invention a method is provided for detecting the presence or absence of variant nucleotides contained within target nucleic acid sequences, said method comprising the following steps:

(i) setting up a reaction mix comprising nucleoside triphosphates or functional derivatives thereof, a polymerizing agent, at least one pair of target-specific amplification primers capable of hybridizing to target nucleic acid sequences, at least one set of labeling primers, each set consisting of at least two types of labeling primers capable of hybridizing to corresponding target nucleic acid sequences 3'-relative to said amplification primers, such that each set of labeling primers is semi-nested relative to the corresponding pair of amplification primers and wherein the semi-nested labelling primers are present in lower concentrations than the pair of amplification primers, the at least two types of labeling primers of a set of labeling primers being characterized by having substantially similar nucleotide sequences except for at least the 3'-terminal nucleotide, which is different for each type of labeling primer and complementary to the variant nucleotide, whereby an extended labeling primer is being synthesized if the 3'-terminal nucleotide of the labeling primer is complementary to the corresponding nucleotide in the target nucleic acid sequence, and whereby substantially no or a negligible background of extended labeling primer is being synthesized when said terminal nucleotide is not complementary to the corresponding nucleotide in the target nucleic acid sequence, and each type of labeling primer further being characterized by carrying a different and distinguishable tag. The reaction mix further comprises at least one sample of target nucleic acid sequences.

(ii) performing an amplification reaction under conditions permitting hybridization of the amplification primers and labeling primers, either together or sequentially, to the corresponding target nucleic acid sequences, and promoting polymerization; and

(iii) monitoring specific properties of said types of labeling primers during or after completion of the amplification reaction, said specific properties being indicative for an extension or non-extension of the labeling primers, thereby detecting the presence or absence of a variant nucleotide contained within a target nucleic acid sequence.

According to the featured method, it should be appreciated that an extension product of the labeling primer is synthesized when the 3'-terminal nucleotide of the labeling primer is complementary to the corresponding nucleotide of the target nucleic acid sequence. It should be further noted that the term "semi-nested", when referring to the labeling primer, means that each type of labeling primer anneals or hybridizes within the region of the target nucleic acid sequence that is framed and amplified by the pair of amplification primers. Each pair of said amplification primers consists of an upstream and corresponding downstream amplification primer, framing the region to be analyzed on the target nucleic acid sequence (see Figure 1 for illustration). The term "target nucleic acid sequence" as used herein is referring to a particular nucleic acid sequence of interest which is suspected of containing, or known to contain a variant nucleotide. The term "variant nucleotide" as used herein is defined as being a nucleotide contained within a target nucleic acid sequence at a defined position which is suspected of being, or known to be, variant. This means that a variant nucleotide may comprise either of four possible nucleotides (adenosine, guanosine, cytidine, and thymidine). In the practice of the invention, for example, a variant nucleotide may comprise a point mutation, deletion, insertion, inversion, or a single nucleotide polymorphism (SNP). Different alleles of a gene may be defined by the presence or absence of a variant nucleotide. It should further be appreciated that a sample of target nucleic acid sequences may contain a mixed population of target nucleic acid molecules, representing at least two different nucleic acid sequences, said target nucleic acid sequences differing at least in their nucleotide composition at the position defined by the corresponding complementary 3'-terminal nucleotide of the labeling primer. Consequently, the reaction mix may contain a mixture of at least two types of extended and amplified labeling primers, said multiple amplified types of labeling primers being distinguishable by virtue of their different tags. In practice, polymorphisms wherein more than two of the four possible nucleotides may be present at a given single-base location, are amenable to qualitative and quantitative analysis by the described method. The reaction mix also comprises nucleoside triphosphates or functional derivatives thereof. As used herein, nucleoside triphosphates or functional derivatives thereof are capable of being incorporated into an extension or amplification product. Functional derivatives of nucleoside triphosphates comprise, for instance, synthetic nucleotides having modified base moieties and/or modified sugar moieties.

In a preferred embodiment the present invention provides a method, as hereinbefore described, wherein the concentration of each labeling primer is 0.1 to 300 times, preferably 5 to 30 times lower than the concentration of the at least one pair of amplification primers. The herein described method preferably employs at least one pair of target nucleic acid sequence-specific amplification primers at high concentrations (e.g. 150 to 300 nM) and at least two types of semi-nested, variant nucleotide-specific labeling primers at significantly lower concentrations (e.g. 1 to 10 nM) in a single amplification reaction, e.g. PCR. During the reaction, the target region of interest is amplified independently of the variant nucleotide sequence, whereas the nested labeling primers are extended with high efficiency only depending on the variant nucleic acid sequence. The 3'-terminal bases of the labeling primers being complementary to the respective variant nucleic acid sequence to be analyzed, there will be an extension of said labeling primer by the polymerase only if the 3'-terminal base of the labeling primer specifically hybridizes with the complementary base in the target nucleic acid sequence. To the contrary, if the 3'-terminal base of the labeling primer is not properly annealed to the target nucleic acid sequence, a specific extension is impaired. Nevertheless, in the case of high DNA concentrations in the amplification reaction an unspecific amplification may occur. In order to minimize the unspecific extension, the semi-nested labeling primers are present in lower concentrations than the pair of amplification primers. Due to their high concentration, the amplification primers dominate the earlier PCR cycles, whereas the low concentrated labeling primers are extended predominantly in later PCR stages, thereby constituting a temporally and spatially semi-nested primer system. This feature has the advantage of leading to high signal-to-noise ratios, very high specificity and minimal danger of obtaining false-positive results.

In another preferred embodiment of the present invention, each labeling primer contains at least one of the following tags: fluorescent dyes, chemiluminescent tags, electroluminescent tags, affinity or binding tags, position specific tags, or tags with specific physical properties such as different size, mass, or gyration.

In a further preferred embodiment of the present invention, the labeling primers contain fluorescent dyes, and each type of labeling primer is tagged with a different fluorescent dye, such that each type of labeling primer is characterized by different excitation- and/or emission spectra, life-time properties, polarization properties, fluorescence resonance enery transfer (FRET) properties, quantum yields, photostability, or triplet number of fluorochromes.

In another preferred embodiment of the inventive method, said tag is permanently or temporarily attached to the labeling primer, preferably to the 5'- end of the labeling primer. The methods of linking, attaching, or conjugating the tag to the labeling primer depend on the type of tag and the position of the tag on the labeling primer and are known in the art. Labeling primers and amplification primers are comprised of oligonucleotides which typically contain 15 to 30 nucleotides. However, depending on the particular complexity of the target sequence, the annealing temperature, and other variable factors, the primers may contain more or fewer nucleotides. The primers may be comprised of phosphodiester oligonucleotides or modified oligonucleotides such as methylphosphonates, phosphotriesters, phosphorothioates, phosphoramidates, PNAs, non-phosphate internucleoside linkages or mixtures of these.

In yet another preferred embodiment of the invention, the amplification reaction and subsequent simultaneous monitoring of the specific properties of the multiple reaction products is performed in a homogeneous format, i.e., in a single reaction tube. It is easily appreciated that this embodiment has the advantage of increased sample through-put and potential cost and time savings.

In a further embodiment of the present method the polymerizing agent is an enzyme. The synthesis of primer extension products can be accomplished, for instance, by E.coli DNA polymerase I, Klenow polymerase, phage T4 DNA polymerase, or other DNA polymerases. A preferred enzyme is a thermostable DNA polymerase. It is particulary desirable to use a thermostable DNA polymerase which lacks 5'- to 3'-exonuclease activity, e.g., a truncated form of *T. aquaticus* DNA polymerase (lawyer et al., *J Biol Chem* 1989, 264:6427-6437; Lawyer et al., *PCR Method Appl* 1993, 2:275-287). By using such an enzyme the labeling primers are not subject to exonuclease degradation from the tag-carrying 5'-terminal end of the labeling primer during the amplification reaction.

In another embodiment of the present invention the target nucleic acid sequences comprise preferably genomic DNA. Additionally, target nucleic acid sequences may comprise cDNA, single stranded DNA, double stranded DNA, plasmid DNA, mixtures of DNA with other molecules. Furthermore, target nucleic acid sequences may be comprised of RNA. When RNA is the starting material for the analysis, a reverse transcription reaction and an amplification reaction may be performed in the same reaction. The origin of the target nucleic acid sequences is preferably human, but it may also originate from other organisms, such as other mammals, vertebrates, invertebrates, fungi, yeast, bacteria, viruses, and plants. The target nucleic acid sequences may be obtained and extracted preferably from blood. It may further be obtained from other accessible tissue material and body fluids such as tissue biopsies, tumor material, skin, hair, sperm, saliva, cord blood, cerebrospinal fluid, and amniotic fluid. Yet another source of target nucleic acid sequences may be from cell culture material. Extraction may be performed by a number of techniques known to those of ordinary skill in the art (see Sambrook and Russell, *Molecular Cloning: A Laboratory Manual,* Cold Spring Harbor Laboratory Press, Cold Spring Harbor, New York, 2000).

In a further preferred embodiment of the invention the monitoring of the specific properties of the amplification reaction products may be determined by a number of different techniques. For instance, chromatographic techniques such as HPLC, FPLC, capillary elecrophoresis, or gel electrophoresis may be employed. Mass spectroscopic or electrochemical techniques may also be used. The monitoring can be performed during (i.e., real time analysis) or after the completion of the PCR-amplification (i.e., end point analysis). Depending on the nature of the labeling tag the invention allows the use of fluorimetric, chromatographic, or physical detection systems to measure the degre of extension of the labeling primers. In the preferred embodiment, fluorimetric analysis is desired for the monitoring of the amplification reaction products. The monitoring can be based on fluorescence polarization analysis, fluorescence anisotropy analysis, fluorescence intensity analysis, fluorescence intensity distribution analysis, fluorescence lifetime analysis, fluorescence dichroism analysis, fluorescence resonance energy transfer analysis, spectroscopic analysis of excitation and/or emission spectra, or, preferably, fluorescence correlation spectroscopy (FCS). It may also be based on combinations of these fluorescence based techniques. Furthermore, fluorimetric analysis may be carried out in a confocal fluorescence system. For multiplex analysis of a homogeneous amplification reaction, fluorescent tags with distinguishable optical parameters such as excitation spectra, emission spectra, fluorescence life-time, polarization, quantum yields, photostability, or triplett number of fluorochromes time are used. In the case of size or mass tags, spectroscopy is a preferred detection system.

In one embodiment of the invention, monitoring the degree of extension of a labeling primer comprises the qualitative detection of variant nucleotides contained within a sample of target nucleic acid sequences. According to this embodiment, an extended labeling primer is indicative for the presence of a variant nucleotide, and a non-extended labeling primer is indicative for the absence of a variant nucleotide.

In another embodiment of the invention monitoring the amounts of extended labeling primers comprises the quantification of target nucleic acid sequences. This feature is particularly useful for genotyping samples of target nucleic acid sequences. According to this embodiment quantification may, for instance, be achieved by (i) calculating the ratio of the values of individually recorded signals (signal A / signal B), or (ii) by subtracting the value of signal B from the value of signal A.

According to one embodiment of the present invention, the method may be used for detecting the presence or absence of one or more variant nucleotides associated with an inherited or acquired condition or disease.

Additionally, according to another embodiment of the present invention, the method may be employed for the diagnostic screening of many samples of target nucleic acid sequences for inherited or acquired conditions or diseases. The method further allows for the screening for the presence or absence of one or more variant nucleotides that may be indicative for a predispositon of acquiring a certain condition or disease.

In a further embodiment of the present invention, the method may be used for quantifying target nucleic acid sequences, in particular for genotyping target nucleic acid sequences. The method may also be useful for the determination of the concentration of DNA or RNA, mRNA, or viral DNA or RNA in a sample. The use of the method for quantification purposes may be preferably desirable for the determination of the copy-number of target nucleic acid sequences, in particular in samples derived from tumor tissue. In the practice of the invention, in order to increase the quantification range of the method, a sample of target nucleic acid sequences may be mixed with a defined amount of engineered internal standard nucleic acids. All of the present nucleic acid sequences are then co-amplified by a polymerase-chain-reaction (PCR) or reverse transcription / polymerase-chain-reaction (RT-PCR) using identical pairs of amplification primers but target- and internal standard-specific labeling primers. The different types of labeling primers within the reaction are distinguished by their tags. The amounts of both types of amplification products are determined by e.g. fluorescence or chromatographic read out. When the amplification kinetic enters the plateau phase the enzymatic amplification efficiency of both targets is reduced. The comparison of the internal standard-specific signal with and without target nucleic acid sequences reflects the amplification efficiency of the total system. A correlation of the amount of amplified products and total amplification efficiency allows for an extension of the quantification range.

In a second aspect, the present invention provides a kit for detecting the presence or absence of variant nucleotides contained within a sample of target nucleic acid sequences in a method according to the invention, said kit comprising: (i) nucleoside triphosphates or functional derivatives thereof, a polymerizing agent, at least one pair of target-specific amplification primers capable of hybridizing to a target nucleic acid sequence, at least one set of labeling primers, each set consisting of at least two types of labeling primers capable of hybridizing to a corresponding target nucleic acid sequence 3'relative to said amplification primers, such that each set of labeling primers is semi-nested relative to the corresponding pair of amplification primers and wherein the semi-nested labelling primers are present in lower concentrations than the pair of amplification primers, the at least two types of labeling primers of a set of labeling primers being characterized by having substantially similar nucleotide sequences except for at least the 3'-terminal nucleotide, which is different for each type of labelling primer and complementary to the variant nucleotide, whereby an extended labeling primer is being synthesized when the 3'-terminal nucleotide of the labeling primer is complementary to the corresponding nucleotide in the target nucleic acid sequence, and each type of labeling primer further being characterized by carrying a different and distinguishable tag. In case the kit of the present invention is preferably used for the quantification of target nucleic acid sequences, it may be desirable to include internal standard nucleic acid sequences. Such internal standards are engineered nucleic acid sequences being substantially identical to an amplified target nucleic acid sequence. In order to ensure comparable enzymatic amplification properties of target nucleic acid sequence and corresponding internal standard nucleic acid sequence, the altered sequence region should be as small as possible, e.g. 1 to 50 nucleotides, preferably 1 to 20, most preferably 1 to 4 nucleotides. In case of longer stretches of altered sequence, the sequence should be scrambled. This means that the nucleic acid sequence is altered in such a way that the overall nucleotide composition (G,C,A,T-content) remains substantially unchanged. In case of DNA target nucleic acid sequences, a DNA internal standard is preferred, in case of RNA target nucleic acid sequences, an RNA internal standard is preferred.

The above and other features and advantages of the invention will be apparent from the following description of figures and examples. The method of the invention is illustrated by means of the example analysis of three SNPs associated with increased risk for deep-vein thrombosis, the $V_{Leiden}$ $G_{1691}A$ transition (ZoUer et al., *J Clin Invest* 1994, 94:2521-2524), the $G_{20210}A$ polymorphism in the prothrombin gene (Poort et al., *Blood* 1996, 88:3698-3703), and the $C_{677}T$ mutation in the gene for 5,10-methylenetetrahydrofolate reductase (MTHFR) (Frosst et al., *Nat Genet* 1995, 10: 111-113).

Example

The specificity, reproducibility, precision, and robustness of the inventive method are demonstrated by the analysis of three SNPs associated with increased risk for deep-vein thrombosis, the factor $V_{Leiden}$ $G_{1691}A$ transition (Bertina et al., *Nature* 1994, 369:64-67; Zoller and Dahlback, *Lancet* 1994, 343:1536-1538; Zoller et al., *J Clin Invest* 1994, 94:2521-2524), the $G_{20210}A$ polymorphism in the prothrombin gene (Degen and Davie, *Biochemistry* 1987, 26: 6165-6177; Martinelli et al., *N Engl J Med* 1998, 338:1793-1797; Poort et al., *Blood* 1996, 88:3698-3703), and the $C_{677}T$ mutation in the gene for 5,10-methylenetetrahydrofolate reductase (MTHFR) (Fodinger et al., *J Nephrol* 2000, 13:20-33; Frosst et al., *Nat Genet* 1995, 10:111-113).

Experimental Methods:

(1) Blood samples and DNA preparation: EDTA blood samples were received from a blood bank and stored at -20°C. Genomic DNA was recovered from whole blood using the QIAamp DNA Blood Mini Kit (QIAGEN, Hilden, Germany) according to the manufacturer's instructions. DNA was quantified by measuring the absorbance at 260 nm and purity of nucleic acids was monitored by controlling the 260 nm / 280 nm absorbance ratios.

(2) Primer design: Amplification and labeling primers were designed using Primer Premier Software, Version 5.00 (Premier Biosoft International, Palo Alto, CA, USA). Default settings in the program were used for reaction conditions, and the following parameters were set for primer selection: primer length = 20 +/- 5 bp; primer melting temperature (Tm) range = 47° - 62°C, primer GC-content = 40 - 65%. Additional parameters were set when necessary to optimize primer selection. Allele-specific labeling primers were positioned with the 3'-terminal base directly at the polymorphic site. Table 1 lists the nucleic acid sequences of the amplification and labeling primers selected for detection of the respective polymorphisms. All primers were synthesized by Interactiva (Ulm, Germany). Labeling primers addressing wildtype alleles were 5'-tagged with TAMRA by standard phosphoramidite chemistry, labeling primers targetted to mutant alleles were linked to N-hydroxysuccinimide-activated EVOblue™50 (EVOTEC Biosystems, Hamburg, Germany) via a C6-linker at the 5'-terminus.

(3) Assay conditions for GALIOS: All PCR reactions were carried out in a final volume of 50 ml using the GeneAmp PCR System 9700 (PE Applied Biosystems, Weiterstadt, Germany). DNA samples were amplified in duplicates and analyzed by FCS directly after cycling without any purification steps. For factor V genotyping, the GALIOS reaction consisted of 1 x amplification buffer [20 mM Tris-HCl, pH 8.9, 10 mM KCl, 10 mM $(NH_4)_2SO_4$, 100 mg/ml BSA, 0.05% Tween-20], 4 mM MgCl2, 0.2 mM of each dNTP, 5% DMSO, 1 U Q-BioTaq (Q-BIOgene, Heidelberg, Germany) and 5 ng genomic DNA. Concentrations of amplification and labeling primers were 300 nM and 10 nM respectively. The following profile was used for thermal cycling: initial 1 min denaturation at 94°C, followed by 40 cycles consisting of 94°C. for 10 sec, 55°C for 10 sec, and 72°C for 10 sec. Samples were maintained at 72°C for 5 min before holding at 4°C prior to analysis. In case of prothrombin genotyping, the GALIOS reaction mixture contained 1 x amplification buffer (same composition as described above), 1.5 mM $MgCl_2$, 0.2 mM of each dNTP, 1 U Q-BioTaq and 10 ng genomic DNA. Amplification and labeling primers were used at concentrations of 300 nM and 5 nM respectively. PCR was performed with the following cycling conditions: 1 min at 94°C, followed by 40 cycles of 94°C for 10 sec, 50°C for 35 sec, and 72°C for 20 sec. Final extension was at 72°C for 5 min. For MTHFR genotyping, the GALIOS reaction mixture contained 1 x amplification buffer (same composition as described above), 1.5 mM $MgCl_2$, 0.2 mM of each dNTP, 1 U Q-BioTaq and 25 ng genomic DNA. Primer concentrations were as in factor V genotyping. Thermal cycling comprised: 1 min at 94°C followed by 40 cycles of 94°C for 25 sec, 55°C for 25 sec, and 72°C for 1 min. Final extension was at 72°C for 5 min.

(4) Assay conditions for PCR allele-specific restriction analysis (PCR-ASRA): For factor V genotyping a PCR reaction was performed in a final volume of 50 ml consisting of: 1 x amplification buffer [20 mM Tris-HCl, pH 8.9, 10 mM KCl, 10 mM $(NH_4)_2SO_4$, 100 mg/ml BSA, 0.05% Tween-20], 4 mM $MgCl_2$, 0.4 mM of each dNTP, 5% DMSO, 300 nM sense primer (5'-GGGCTAATAGGACTACTTCTAATC-3'), 300 nM antisense primer (5'-AGCCAGGAGACCTAACAT-3'), 1 U Q-BioTaq and 300 ng genomic DNA. PCR amplification was achieved using the following cycling conditions: initial 5 min denaturation at 94°C followed by 40 cycles consisting of 94°C for 45 sec, 55°C for 45 sec, and 72°C for 30 sec. Finally, an elongation step at 72°C for 5 min was performed. The amplified 135 bp PCR product was purified by ethanol precipitation and digested with 10 U *MnI*I (New England Biolabs GmbH, Frankfurt, Germany) at 37°C for 1.5 hours. Digested samples were analyzed by electrophoresis in a 3% TAE-agarose gel stained with ethidium bromide. A 100-bp DNA ladder (Life Technologies, Karlsruhe, Germany) was used as size marker.

(5) Data acquisition and analysis: After thermal cycling, 5 ml of each PCR reaction mix analyzed by agarose-gel electrophoresis to monitor successful PCR amplification. 20 ml of each PCR reaction mix were transferred to 96-well microplates (UniView™, Whatman via Merck Eurolab, Bochum, Germany) without any post-PCR-modification. FCS measurements were performed on a modified confocal microscope Olympus IX50 (Olympus, Hamburg, Germany) equipped with standard filter sets for excitation with a helium-neon laser (either 543 or 632 nm line, 40 mW, Uniphase), UApo 40×/1.15W objective, pinhole 50 mM. Each PCR reaction mix was measured 5 times, 4 seconds each, at both 543 nm and 632 nm. From the photon count signal, the autocorrelation curve was calculated and evaluated with the autocorrelation function G(t) using a two-component model corresponding to free and extended primer (Schwille et al., *Biophys J* 1997, 72:1878-1886):

$$G(\tau) = \left[1 - T + T\exp\left(\frac{-\tau}{\tau_T}\right)\right] N^{-1} \left[\frac{1-Y}{\left(1+\frac{\tau}{\tau_{free}}\right)\sqrt{1+\frac{r_0^2}{z_0^2}\frac{\tau}{\tau_{free}}}} + \frac{Y}{\left(1+\frac{\tau}{\tau_{bound}}\right)\sqrt{1+\frac{r_0^2}{z_0^2}\frac{\tau}{\tau_{bound}}}}\right]$$

where T is the average fraction of dye molecules in the triplet state with relaxation time t; N is the total average number of fluorescent molecules in the observation volume; Y is the relative fraction of extended primer; $t_{free}$ and $t_{poly}$ define the average diffusion times for free primers and extended primers (i.e., the PCR product), respectively, through the confocal observation volume. The parameter S = $r_0/z_0$ describes the ratio of the radius to the length of the detection area, where $r_0$ and $z_0$ are lateral and axial radii defining the football-like confocal observation volume (i.e., the distances between the coordinate where the Gaussian distribution of the emission light reaches its maximum value and the point where the light intensity decreases to $1/e^2$ of this maximum value). FCS data evaluation was performed with the the FCS+plus software (Version 1.00, EVOTEC BioSystems, Hamburg, Germany) and provided translational diffusion times and relative fractions of fluorescent primers and PCR products. The relative fraction of PCR product from 10 measurements (resulting from duplicate PCR reactions, each measured 5 times) was calculated to means +/- standard deviation (SD). This was done separately for measurements obtained at 543 nm or 632 nm. Finally, the 543/632 ration was calculated from the corresponding means of each (duplicate) probe. The standard deviation ($SD_{x,y}$) of the 543/632 ratio was calculated according to:

$$SD_{x,y} = \sqrt{\left(\frac{1}{y}\right)^2 \times SD_x^{\,2} + \left(\frac{-x}{y^2}\right)^2 \times SD_y^{\,2}}$$

with X(Y) representing means at 543 (632) nm and $SD_x$ ($SD_y$) representing the corresponding SD. 95% confidence intervals of the 543/632 ratios were calculated as:

$$543/632 \pm 1.96 \times \left(\frac{SD_{x,y}}{\sqrt{10}}\right)$$

Results and Analysis:

The applicability of the method of the invention (GALIOS) for determining the presence or absence of variant nucleotides was examined by comparing it with an established method for genotyping. A sample of genomic target nucleic acid sequences with known allelic constitutions at the $G_{1691}A$ polymorphic site (i.e. the variant nucleotide) of the factor V gene (wt: G/G; hz: G/A; mut: A/A) was examined in parallel by (1) conventional PCR amplification and subsequent allele-specific restriction analysis (PCR-ASRA) and by (2) the GALIOS method. For PCR-ASRA, a 135 bp region containing the $G_{1691}A$ polymorphism of the factor V gene was amplified from the target nucleic acid sequences and was subsequently digested with *MnlI* (Figure 3A,B). Because the $G_{1691}A$ transition destroys an *MnlI* restriction site in the factor V gene, the amplification product obtained from the mutant target nucleic acid sequences remained undigested while in the case of wildtype target nucleic acid sequences, the amplification product was cut into a 43 bp and a 92 bp fragment. *MnlI* digestion of the amplification product obtained from heterozygous target nucleic acid sequences yielded three DNA fragments of 43 bp, 92 bp and 135 bp. For genotyping by the GALIOS method, the same samples of genomic target nucleic acid sequences and a negative control (containing no target nucleic acid sequences) were amplified in duplicates by the GALIOS method. After 40 thermal cycles, the reaction mixes were analyzed without any post-PCR processing, physical separation, or dilution steps by fluorescence correlation spectroscopy (Figure 3C). The volume of the detection field at 543 nm and 632 nm was determined by measuring 5 nM TAMRA and 5 nM EVOblue™50, respectively. The ratio of the radius to the length of the confocal detection volume, S, was 6.9 at 543 nm and 3.4 at 632 nm. The translational diffusion time of the free labeling primers, $\tau_{free}$, defined from the GALIOS negative control reaction was 0.153 ms at 543 nm and 0.342 ms at 632 nm. The translational diffusion time of the 314 bp fluorescent amplification product, $\tau_{poly}$, was evaluated by curve fitting of the autocorrelation data with fixed values for S and $\tau_{free}$ and yielded 1.348 ms at 543 nm and 2.073 ms at 632 nm. Because the translational diffusion times of non-extended primers and amplified products were clearly distinguishable at both 543 nm and 632 nm, it was possible to quantify the fractions of the amplified products. All samples were measured 5 times, for 4 seconds each, at 543 nm and 632 nm. The values of S, $\tau_{free}$, and $\tau_{poly}$ were fixed in the fitting algorithm as described above to reduce the numbers of free parameters and to quantify the fractions of the amplified products. Means $\pm$ SD of the relative fractions of fluorescent amplification product were calculated for each genotype. The reactions containing factor V wildtype target nucleic acid sequences produced 42.85 $\pm$ 1.77 % amplification product at 543 nm, but only 4.14 $\pm$ 0.61 % amplification product at 632 nm. Heterozygous target nucleic acid sequences for the factor V $G_{1691}A$ polymorphism yielded significant amplification product fractions at both wavelengths, 31.50 $\pm$ 1.30 % at 543 nm and 52.40 $\pm$ 1.10 % at 632 nm. Target nucleic acid sequences containing only the mutant factor $V_{Leiden}$ allele had amplification product values of only 2.52 $\pm$ 0.99 % at 543 nm, but 61.95 $\pm$ 0.29 % at 632 nm. The control reactions which contained no target nucleic acid sequences had only very low background amplification product fractions at both wavelengths: 1.83 $\pm$ 0.11 % at 543 nm and 1.24 $\pm$ 0.94 % at 632 nm. In all cases, amplification product values indicating allele-specific primer extension were at least 15-fold higher than the corresponding background noise at the respective wavelength. By calculation of the 543/632 ratios of the amplification product values from each duplicate sample, representative values for each genotype (Figure 3D) were obtained. Overall, the genotyping results obtained by the method of the invention were comparable to the results obtained by the PCR-ASRA genotyping procedure. To further evaluate the specificity of the method of invention, 18 samples of target nucleic acid sequences with known factor V genotypes, six samples for each genotype, were amplified in duplicates and analyzed as described above. All of the 18 samples were assigned a correct genotype. False-positive or false-negative genotype determinations were not observed. Figure 4A illustrates means $\pm$ SD of relative amplification product fractions for each genotype: wildtype (543 nm): 44.79 $\pm$ 4.0 %; wildtype (632 nm) 4.22 $\pm$ 3.03 %; heterozygous (543 nm): 33.04 $\pm$ 4.55 %; heterozygous (632 nm) 41.98 $\pm$ 4.88 %; mutant (543 nm): 4.09 $\pm$ 2.38 %; mutant (632 nm) 64.05 $\pm$ 4.63 %. The mean 543/632 ratios $\pm$ SD of the amplification product fractions were again distinct for each genotype: 10.61 $\pm$ 5,13 for wildtype, 0.79 $\pm$ 0.08 for heterozygous and 0.06 $\pm$ 0.04 for mutant samples of target nucleic acid sequences.

Reproducibility and precision of the method of invention were examined by analyzing each of the three different factor V genotypes in 36 independent experiments: 18 samples of target nucleic acid sequences, (six samples for each genotype) were analyzed in parallel by three different experimentators on two following days. The total test set comprised 108 individual reactions, and each individual sample of target nucleic acid sequences was genotyped six times. All of the reactions resulted in correct genotype determination. These results underline the very high reproducibility (100 %) of genotyping by the method of invention. Figure 4B lists the mean 543/632 ratios $\pm$ SD for each genotype. At 95 % confidence, the 543/632 ratios for factor V wildtype, heterozygous and mutant samples range from 9.49 -12.49, 0.63 - 0.67 and 0.03 - 0.05, respectively. These data show that although the assays were carried out on different days, by different experimentators, and with different samples of target nucleic acid sequences for a given genotype, the obtained results were highly reproducible and precise.

Furthermore, the specificity of the method of invention was examined by analyzing 9 genomic samples of target nucleic acid sequences (3 samples for each factor V genotype) in the presence of a ~250-fold molar excess of genomic DNA from *Saccharomyces cerevisiae* (Figure 5). The genotyping results were correct in all cases and yielded values practically identical to those obtained from control reactions without S. *cerevisiae* DNA. The data demonstrated that the method of invention is highly specific and provides unambiguous results even in the presence of a high background of contaminating DNA.
In another set of experiments, the robustness of genotyping by the method of invention upon addition of varying amounts of target nucleic acid sequences was examined. Three samples for each factor V genotype were analyzed by the method of invention. The amount of target nucleic acid sequences varied from 2.5 to 7.5 ng per assay. As shown in Figure 6, changing the total amount of target nucleic acid sequences by ± 50% did not have significant effects on the quality and quantity of the fluorescence signal.
In order to verify that the method of invention is generally suited for single nucleotide polymorphism (SNP) detection, two additional SNPs which, like the factor $V_{Leiden}$ $G_{1691}A$ polymorphism, have been reported to play a role in the predisposition to deep-vein thrombosis, were analyzed: the $G_{20210}A$ polymorphism in the prothrombin gene and the $C_{677}T$ mutation in the gene for 5,10-methylenetetrahydrofolate reductase (MTHFR).
For both SNPs, 18 samples of target nucleic acid sequences with known genotypes, (six samples for each genotype) were amplified and analyzed according to the experimental strategy described above for factor V. All of the 36 genotypes were assigned correctly with no false-positive or false-negative determinations. Figure 7 (A,C) illustrates means ± SD of relative amplification product fractions for each genotype of prothrombin and MTHFR, respectively. The reproducibility and precision of prothrombin- and MTHFR genotyping by the method of invention were analyzed by the same experimental setup as described above for factor V: each genotype of each polymorphism was examined independently 36 times, with six individual determinations for each sample of target nucleic acid sequences. All of the 216 reactions provided correct SNP scoring. At 95 % confidence, the values for wildtype, heterozygous and mutant samples ranged from 9.77 - 14.57, 1.00 - 1.12, and 0.10 - 0.14 for prothrombin and from 10.96 -15.50, 0.96 - 1.04, and 0.10 - 0.11 for MTHFR, respectively (Figure 7B,D).

Description of Figures:

Figure 1 illustrates one embodiment of the principle of the inventive GALIOS method for the analysis of variant nucleic acid sequences. Gene specific amplification primers (F,R) amplify wildtype and mutant alleles with equal efficiency. Allele-specific labeling primers (L1,L2) are attached to different and distinguishable fluorescent tags and simultaneously amplify efficiently only the allele with the corresponding variant nucleotide. This results in allele-specific accumulation of fluorescent amplification products detectable preferably by fluorescent correlation spectroscopy.
Figure 2 depicts the read out of the analysis of the factor V polymorphism by GALIOS using fluorescence correlation spectroscopy (FCS). A) The relative amounts of the two labeling primers and their extension products are determined by the present method. The three factor V genotypes (AA, AB, BB) are represented on the x-axis, and the corresponding amount of extension product (fluorescence signal in relative units) are shown on the y-axis. The black bars show the TAMRA-labeled products (excited at 543 nm), and the hatched bars show the EVOblue™ 50-labeled products (excited at 632 nm) amplified in the same reaction. B) For the purpose of more convenient genotyping, the measured fluorescence signals for the TAMRA-labeled molecules are divided by the fluorescence signal for the EVOblue™50-labeled molecules. The obtained values allow for unambiguous and highly specific genotyping.
Figure 3 illustrates the analysis of the $G_{1691}A$ polymorphism of the factor V gene by conventional amplification followed by allele-specific restriction analysis (PCR-ASRA) and by the method of the invention (GALIOS). A) Schematic illustration of the Mnl I restriction digestion. A 135 bp amplification product from the wildtype allele is cut into a 43 bp and a 92 bp fragment while the amplification product obtained from the mutant allele remains undigested. B) Analysis of Mnl I restriction by agarose gel electrophoresis. Lanes 1,8: 100 bp DNA ladder. Lanes 2-7: PCR products from genomic DNA being wildtype, wt (2,3), heterozygous, hz (4,5) or mutant, mut (6,7) for the factor V $G_{1691}A$ polymorphism before (2,4,6) and after (3,5,7) Mnl I digestion. All samples yielded the expected restriction fragment patterns, i.e., 92 bp and 43 bp for wildtype; 135 bp, 92 bp and 43 bp for heterozygous; and 135 bp for mutant DNA. C) GALIOS genotyping of the factor V $G_{1691}A$ polymorphism. Means +/-SD of relative fractions of fluorescent PCR product obtained at 543 nm and 632 nm for each factor V genotype (wt, hz, mut) and for a negative control without template (n.c.). D) Mean 543/632 ratios +/- SD and corresponding 95% confidence intervals for each factor V genotype.
Figure 4 shows data for the specificity, reproducibility and precision of the method of invention. A) Six genomic DNA samples were analyzed for each factor V genotype. The graph shows means +/- SD of relative fractions of fluorescent amplification products obtained at 543 nm and 632 nm for each genotype. B) Mean 543/632 ratios +/- SD and corresponding 95% confidence intervals for each factor V genotype are listed. Data were obtained from n = 36 independent determinations of each genotype.
Figure 5 depicts the specificity of the GALIOS method in the presence of contaminating template. GALIOS genotyping was performed in parallel with human genomic DNA and with a mixture of human genomic DNA and contaminating *S. cerevisiae* DNA (5 ng each). This represents a 250-fold molar excess of *S. cerevisiae* DNA over human genomic DNA. Three genomic DNA samples for each genotype were tested. Data represent means +/- SD of fluorescent signals for each genotype.

Figure 6 exemplifies the robustness of the GAUOS method of determining the presence or absence of variant nucleotides. The amount of target nucleic acid sequences in the assay was varied by +/- 50% of the optimal concentration (5 ng). Three genomic DNA samples for each genotype were analyzed. Data represent means +/- SD of fluorescent signals for each genotype.

Figure 7 shows the results of GALIOS genotyping of the prothrombin $G_{20210}A$ and the MTHFR $C_{677}T$ polymorphisms. A,C) Six genomic DNA samples for each prothrombin (A) or for each MTHFR (C) genotype were analyzed in duplicates. The graphs show means +/- SD of relative fractions of fluorescent PCR products obtained at 543 nm and 632 nm. B,D) Mean 543/632 ratios +/- SD and corresponding 95% confidence intervals for each prothrombin (B) and MTHFR (D) genotype are listed. Data were obtained from n = 36 independent determinations of each genotype.

## Claims

**1.** A method of detecting the presence or absence of variant nucleotides contained within target nucleic acid sequences, said method comprising the following steps:

(i) setting up a reaction mix comprising:

- nucleoside triphosphates, or functional derivatives thereof
- a polymerizing agent
- at least one pair of target-specific amplification primers capable of hybridizing to target nucleic acid sequences
- at least one set of labelling primers, each set consisting of at least two types of labelling primers capable of hybridizing to corresponding target nucleic acid sequences 3'-relative to said amplification primers, such that each set of labelling primers is semi-nested relative to the corresponding pair of amplification primers, and wherein the semi-nested labelling primers are present in lower concentrations than the pair of amplification primers,
the at least two types of labelling primers of a set of labelling primers being **characterized by** having substantially similar nucleotide sequences except for at least the 3'-terminal nucleotide, which is different for each type of labelling primer and complementary to the variant nucleotide, whereby an extended labelling primer is being synthesized if the 3'-terminal nucleotide of the labelling primer is complementary to the corresponding nucleotide in the target nucleic acid sequence, and whereby substantially no or a negligible background of extended labelling primer is being synthesized if said terminal nucleotide is not complementary to the corresponding nucleotide in the target nucleic acid sequence and each type of labelling primer further being **characterized by** carrying a different and distinguishable tag; and
- at least one sample of target nucleic acid sequences

(ii) performing an amplification reaction under conditions permitting hybridization of the amplification primers and labelling primers, either together or sequentially, to the corresponding target nucleic acid sequences, and promoting polymerization; and
(iii) monitoring specific properties of said types of labelling primers during or after completion of the amplification reaction, said specific properties being indicative for an extension or non-extension of the labelling primers, thereby detecting the presence or absence of a variant nucleotide contained within a target nucleic acid sequence.

2. The method according to claim 1 wherein the concentration of each labelling primer is 0.1 to 300 times, preferably 5 to 30 times lower than the concentration of each amplification primer,

3. The method according to claims 1 and 2 wherein each labelling primer contains at least one tag selected from the group consisting of fluorescent dyes, chemiluminescent tags, electroluminescent tags, affinity or binding tags, position specific tags, and/or tags with specific physical properties such as different size, mass, or gyration.

4. The method according to claims 1 to 3 wherein the labelling primers contain fluorescent dyes, and wherein each type of labelling primer is tagged with a different fluorescent dye, such that each type of labelling primer is **characterized by** different excitation- and/or emission spectra, life-time properties, polarization properties, fluorescence-resonance-energy-transfer (FRET) properties, quantum yields, photostability, or triplet number of fluorochromes.

5. The method according to claims 1 to 4 wherein the tag is permanently or temporarily attached to the labelling primer, preferably to the 5'-end of the labelling primer.

6. The method according to claims 1 to 5 wherein the amplification reaction and monitoring are performed in a homogeneous format.

7. The method according to claims 1 to 6 wherein the polymerizing agent is an enzyme, preferably a DNA polymerase, preferably a thermostable DNA polymerase, most preferably a thermostable DNA polymerase which lacks 5'-to 3'-exonuclease activity.

8. The method according to claims 1 to 7 wherein a sample of target nucleic acid sequences is selected from a group comprising genomic DNA, cDNA, single stranded DNA, double stranded DNA, plasmid DNA, RNA, mixtures of DNA with other molecules, DNA or RNA from human sources or other sources such as mammals, vertebrates, invertebrates, bacteria, viruses, yeast, fungi, or plants.

9. The method according to claims 1 to 8 wherein the monitoring of the specific properties of said amplification reaction products is determined by chromatographic techniques such as HPLC, FPLC, capillary electrophoresis, gel electrophoresis, or by mass spectroscopic or electrochemical techniques, or preferably by fluorescent techniques such as fluorescent polarization spectroscopy, fluorescent life-time spectroscopy, fluorescence intensity distribution analysis (FIDA), fluorescence dichroism analysis, fluorescence intensity analysis, fluorescence resonance energy transfer (FRET) analysis, spectroscopic analysis of excitation and/or emission spectra, or, in particular, by fluorescence correlation spectroscopy.

10. The method according to claims 1 to 9 wherein monitoring the degree of extension of a labelling primer comprises the qualitative detection of variant nucleotides contained within a sample of target nucleic acid sequences, insofar as an extended labelling primer is indicative for the presence of a variant nucleotide and a non-extended labelling primer is indicative for the absence of a variant nucleotide.

11. The method according to claims 1 to 9 wherein monitoring the amount of extended labelling primers comprises the quantification of target nucleic acid sequences, in particular the genotyping of samples of target nucleic acid sequences, preferably by (i) calculating the ratio of the values of individually recorded signals (signal A/ signal B), or (ii) by subtracting the value of signal B from the value of signal A.

12. Use of the method according to claims 1 to 11 for detecting the presence or absence of one or more variant nucleotides associated with an inherited or acquired condition or disease.

13. Use of the method according to claims 1 to 11 for diagnostic screening of samples of target nucleic acid sequences for inherited or acquired conditions and diseases, and for screening for predispositions to such conditions and diseases.

**14.** Use of the method according to claims 1 to 11 for quantifying target nucleic acid sequences, in particular for genotyping target nucleic acid sequences, determining the concentration of DNA, or RNA, or mRNA, or viral RNA in a sample, or determining the copy-number of target nucleic acid sequences, particularly determining the copy-number of target nucleic acid sequences in samples derived from tumor tissue.

**15.** A kit for detecting the presence or absence of variant nucleotides contained within a sample of target nucleic acid sequences in a method according to claims 1-11, said kit comprising:

- nucleoside triphosphates, or functional derivatives thereof
- a polymerizing agent
- at least one pair of target-specific amplification primers capable of hybridizing to target nucleic acid sequences
- at least one set of labelling primers, each set consisting of at least two types of labelling primers capable of hybridizing to corresponding target nucleic acid sequences 3'-relative to said amplification primers, such that each set of labelling primers is semi-nested relative to the corresponding pair of amplification primers, and wherein the semi-nested labelling primers are present in lower concentrations than the pair of amplification primers,

the at least two types of labelling primers of a set of labelling primers being **characterized by** having substantially similar nucleotide sequences except for at least the 3'-terminal nucleotide, which is different for each type of labelling primer and complementary to the variant nucleotide, whereby an extended labelling primer is being synthesized if the 3'-terminal nucleotide of the labelling primer is complementary to the corresponding nucleotide in the target nucleic acid sequence, and whereby substantially no or a negligible background of extended labelling primer is being synthesized if said terminal nucleotide is not complementary to the corresponding nucleotide in the target nucleic acid sequence and each type of labelling primer further being **characterized by** carrying a different and distinguishable tag.

**16.** The kit according to claim 15 comprising at least one sample of internal standard nucleic acid sequences.

**Patentansprüche**

**1.** Verfahren zum Nachweisen der Anwesenheit oder Abwesenheit von abweichenden Nucleotiden innerhalb von Zielnucleinsäuresequenzen, wobei das Verfahren die folgenden Schritte umfasst:

(i) Herstellen eines Reaktionsgemischs, umfassend:

- Nucleosidtriphosphate oder funktionelle Derivate davon;
- ein Polymerisationsmittel;
- wenigstens ein Paar von zielspezifischen Amplifikationsprimern, die mit Zielnucleinsäuresequenzen hybridisieren können;
- wenigstens ein Satz Markierungsprimer, wobei jeder Satz aus wenigstens zwei Typen von Markierungsprimern besteht, die mit entsprechenden Zielnucleinsäuresequenzen 3' von den Amplifikationsprimern hybridisieren können, so dass jeder Satz Markierungsprimer relativ zu dem entsprechenden Paar von Amplifikationsprimern halbgeschachtelt (semi-nested) ist, und wobei die halbgeschachtelten Markierungsprimer in niedrigeren Konzentrationen vorhanden sind als das Paar von Amplifikationsprimern;

wobei die wenigstens zwei Typen von Markierungsprimern eines Satzes Markierungsprimer **dadurch gekennzeichnet sind, dass** sie im Wesentlichen ähnliche Nucleotidsequenzen haben, abgesehen von wenigstens dem 3'-terminalen Nucleotid, das für jeden Typ von Markierungsprimer verschieden und zu dem abweichenden Nucleotid komplementär ist, wodurch ein verlängerter Markierungsprimer synthetisiert wird, wenn das 3'-terminale Nucleotid des Markierungsprimers komplementär zu dem entsprechenden Nucleotid in der Zielnucleinsäuresequenz ist, und wodurch im Wesentlichen kein oder nur ein vernachlässigbarer Hintergrund von verlängertem Markierungsprimer synthetisiert wird, wenn das terminale Nucleotid nicht komplementär zu dem entsprechenden Nucleotid in der Zielnucleinsäuresequenz ist, und jeder Typ von Markierungsprimer weiterhin **dadurch gekennzeichnet ist, dass** er eine andere und unterscheidbare Markierung trägt; und

- wenigstens eine Probe von Zielnucleinsäuresequenzen;

(ii) Durchführen einer Amplifikationsreaktion unter Bedingungen, die die- Hybridisierung der Amplifikationsprimer und der Markierungsprimer, entweder zusammen oder nacheinander, an die entsprechenden Zielnucleinsäuresequenzen ermöglichen, und Fördern der Polymerisation; und

(iii) Messen spezieller Eigenschaften der Typen von Markierungsprimern während oder nach der Beendigung der Amplifikationsreaktion, wobei die spezifischen Eigenschaften auf eine Verlängerung oder Nichtverlängerung der Markierungsprimer hinweisen, wodurch die Anwesenheit oder Abwesenheit eines abweichenden Nucleotids innerhalb einer Zielnucleinsäuresequenz nachgewiesen wird.

**2.** Verfahren gemäß Anspruch 1, wobei die Konzentration jedes Markierungsprimers 0,1- bis 300mal, vorzugsweise 5- bis 30mal, niedriger ist als die Konzentration jedes Amplifikationsprimers.

**3.** Verfahren gemäß Anspruch 1 und 2, wobei jeder Markierungsprimer wenigstens eine Markierung enthält, die aus der Gruppe ausgewählt ist, die aus Fluoreszenzfarbstoffen, Chemilumineszenzmarkern, Elektrolumineszenzmarkern, Affinitäts- oder Bindungsmarkern, positionsspezifischen Markern und/oder Markern mit spezifischen physikalischen Eigenschaften, wie unterschiedlicher Größe, Masse oder Drehrichtung, besteht.

**4.** Verfahren gemäß Anspruch 1 bis 3, wobei die Markierungsprimer Fluoreszenzfarbstoffe enthalten und wobei jeder Typ von Markierungsprimer mit einem anderen Fluoreszenzfarbstoff markiert ist, so dass jeder Typ von Markierungsprimer durch unterschiedliche Anregungs- und/oder Emissionsspektren, Lebensdauereigenschaften, Polarisationseigenschaften, Fluoreszenz-Resonanz-Energie-Transfer(FRET)-Eigenschaften, Quantenausbeuten, Photostabilität oder Triplettzahl der Fluorochrome **gekennzeichnet** ist.

**5.** Verfahren gemäß Anspruch 1 bis 4, wobei der Marker permanent oder temporär an den Markierungsprimer, vorzugsweise das 5'-Ende des Markierungsprimers, gebunden ist.

**6.** Verfahren gemäß Anspruch 1 bis 5, wobei die Amplifikationsreaktion und das Messen in einem homogenen Format durchgeführt werden.

**7.** Verfahren gemäß den Ansprüchen 1 bis 6, wobei das Polymerisationsmittel ein Enzym ist, vorzugsweise eine DNA-Polymerase, vorzugsweise eine thermostabile DNA-Polymerase, am meisten bevorzugt eine thermostabile DNA-Polymerase, der die 5'$\rightarrow$3'-Exonuclease-Aktivität fehlt.

**8.** Verfahren gemäß den Ansprüchen 1 bis 7, wobei die Probe von Zielnucleinsäuresequenzen aus einer Gruppe ausgewählt ist, die aus genomischer DNA, cDNA, einzelsträngiger DNA, doppelsträngiger DNA, Plasmid-DNA, RNA, Gemischen von DNA mit anderen Molekülen, DNA oder RNA aus menschlichen Quellen oder anderen Quellen, wie Säugern, Wirbeltieren, Wirbellosen, Bakterien, Viren, Hefen, Pilzen oder Pflanzen, besteht.

**9.** Verfahren gemäß den Ansprüchen 1 bis 8, wobei das Messen der speziellen Eigenschaften der Amplifikationsreaktionsprodukte durch chromatographische Techniken, wie HPLC, FPLC, Kapillarelektrophorese, Gelelektrophorese, oder durch massenspektroskopische oder elektrochemische Techniken oder vorzugsweise durch Fluoreszenztechniken, wie Fluoreszenzpolarisationsspektroskopie, Fluoreszenzlebensdauerspektroskopie, Fluoreszenzintensitätsverteilungsanalyse (FIDA), Fluoreszenzdichroismusanalyse, Fluoreszenzintensitätsanalyse, Fluoreszenz-Resonanz-EnergieTransfer(FRET)-Analyse, spektroskopische Analyse von Anregungs- und/oder Emissionsspektren oder insbesondere durch Fluoreszenzkorrelationsspektroskopie erfolgt.

**10.** Verfahren gemäß den Ansprüchen 1 bis 9, wobei das Messen des Verlängerungsgrads eines Markierungsprimers insofern den qualitativen Nachweis von abweichenden Nucleotiden innerhalb einer Probe von Zielnucleinsäuresequenzen umfasst, als ein verlängerter Markierungsprimer die Anwesenheit eines abweichenden Nucleotids anzeigt und ein nicht verlängerter Markierungsprimer die Abwesenheit eines abweichenden Nucleotids anzeigt.

**11.** Verfahren gemäß den Ansprüchen 1 bis 9, wobei das Messen der Menge der verlängerten Markierungsprimer die Quantifizierung von Zielnucleinsäuresequenzen, insbesondere die Genotypbestimmung von Proben von Zielnucleinsäuresequenzen, umfasst, vorzugsweise (i) durch Berechnen des Verhältnisses der Werte von individuell aufgezeichneten Signalen (Signal A/Signal B) oder (ii) durch Subtrahieren des Werts von Signal B von dem Wert von Signal A.

**12.** Verwendung des Verfahrens gemäß den Ansprüchen 1 bis 11 zum Nachweis der Anwesenheit oder Abwesenheit von einem oder mehreren abweichenden Nucleotiden, die mit einem ererbten oder erworbenen Zustand bzw. Krankheit assoziiert sind.

**13.** Verwendung des Verfahrens gemäß den Ansprüchen 1 bis 11 zum diagnostischen Durchmustern von Proben von Zielnucleinsäuresequenzen auf ererbte oder erworbene Zustände und Krankheiten und zum Durchmustern auf Prädispositionen für solche Zustände und Krankheiten.

**14.** Verwendung des Verfahrens gemäß den Ansprüchen 1 bis 11 zum Quantifizieren von Zielnucleinsäuresequenzen, insbesondere zur Genotypbestimmung von Zielnucleinsäuresequenzen, zum Bestimmen der Konzentration von DNA oder RNA oder mRNA oder viraler RNA in einer Probe oder zum Bestimmen der Kopienzahl von Zielnucleinsäuresequenzen, insbesondere zum Bestimmen der Kopienzahl von Zielnucleinsäuresequenzen in Proben, die von Tumorgewebe abgeleitet sind.

**15.** Kit zum Nachweisen der Anwesenheit oder Abwesenheit von abweichenden Nucleotiden innerhalb einer Probe von Zielnucleinsäuresequenzen in einem Verfahren gemäß den Ansprüchen 1 bis 11, wobei der Kit Folgendes umfasst:

- Nucleosidtriphosphate oder funktionelle Derivate davon;
- ein Polymerisationsmittel;
- wenigstens ein Paar von zielspezifischen Amplifikationsprimern, die mit Zielnucleinsäuresequenzen hybridisieren können;
- wenigstens ein Satz Markierungsprimer, wobei jeder Satz aus wenigstens zwei Typen von Markierungsprimern besteht, die mit entsprechenden Zielnucleinsäuresequenzen 3' von den Amplifikationsprimern hybridisieren können, so dass jeder Satz Markierungsprimer relativ zu dem entsprechenden Paar von Amplifikationsprimern halbgeschachtelt (semi-nested) ist, und wobei die halbgeschachtelten Markierungsprimer in niedrigeren Konzentrationen vorhanden sind als das Paar von Amplifikationsprimern;

wobei die wenigstens zwei Typen von Markierungsprimern eines Satzes Markierungsprimer **dadurch gekennzeichnet sind, dass** sie im Wesentlichen ähnliche Nucleotidsequenzen haben, abgesehen von wenigstens dem 3'-terminalen Nucleotid, das für jeden Typ von Markierungsprimer verschieden und zu dem abweichenden Nucleotid komplementär ist, wodurch ein verlängerter Markierungsprimer synthetisiert wird, wenn das 3'-terminale Nucleotid des Markierungsprimers komplementär zu dem entsprechenden Nucleotid in der Zielnucleinsäuresequenz ist, und wodurch im Wesentlichen kein oder nur ein vernachlässigbarer Hintergrund von verlängertem Markierungsprimer synthetisiert wird, wenn das terminale Nucleotid nicht komplementär zu dem entsprechenden Nucleotid in der Zielnucleinsäuresequenz ist, und jeder Typ von Markierungsprimer weiterhin **dadurch gekennzeichnet ist, dass** er eine andere und unterscheidbare Markierung trägt.

**16.** Kit gemäß Anspruch 15, der wenigstens eine Probe von internen Standardnucleinsäuresequenzen umfasst.

## Revendications

**1.** Procédé de détection de la présence ou de l'absence de nucléotides variants contenus dans des séquences d'acide nucléique cibles, ledit procédé comprenant les étapes suivantes consistant à :

(i) élaborer un mélange réactionnel comprenant :

- des nucléoside triphosphates, ou des dérivés fonctionnels de ceux-ci
- un agent polymérisant
- au moins une paire d'amorces d'amplification spécifiques d'une cible capables de s'hybrider sur des séquences d'acide nucléique cibles
- au moins un jeu d'amorces de marquage, chaque jeu étant constitué d'au moins deux types d'amorces de marquage capables de s'hybrider sur des séquences d'acides nucléiques cibles correspondantes côté 3' par rapport auxdites amorces d'amplification, de sorte que chaque jeu d'amorces de marquage est semi-niché par rapport à la paire correspondante d'amorces d'amplification, et dans lequel les amorces de marquage semi-nichées sont présentes à des concentrations inférieures à celles de la paire d'amorces d'amplification,

les au moins deux types d'amorces de marquage d'un jeu d'amorces de marquage étant **caractérisés en ce qu'**ils ont des séquences de nucléotides essentiellement similaires à l'exception d'au moins le nucléotide 3'-terminal, qui est différent pour chaque type d'amorce de marquage et complémentaire du nucléotide variant, si bien qu'une amorce de marquage allongée est synthétisée si le nucléotide 3'-terminal de l'amorce de marquage est complémentaire du nucléotide correspondant dans la séquence d'acides nucléiques cible, et si bien qu'essentiellement aucun ou un bruit de fond négligeable d'amorce de marquage allongée n'est ou est synthétisée si ledit nucléotide terminal n'est pas complémentaire du nucléotide correspondant dans la séquence d'acide nucléique cible et chaque type d'amorce de marquage étant en outre **caractérisé en ce qu'**il porte un marqueur différent et pouvant être différencié ; et

• au moins un échantillon de séquences d'acide nucléique cibles

(ii) réaliser une réaction d'amplification dans des conditions permettant l'hybridation des amorces d'amplification et des amorces de marquage, soit simultanément soit successivement, sur les séquences d'acide nucléique cibles correspondantes, et activer la polymérisation ; et

(iii) contrôler les propriétés spécifiques desdits types d'amorces de marquage pendant ou après l'achèvement de la réaction d'amplification, lesdites propriétés spécifiques étant indicatrices d'un allongement ou d'un non-allongement des amorces de marquage, détectant ainsi la présence ou l'absence d'un nucléotide variant présent dans une séquence d'acides nucléiques cible.

**2.** Procédé selon la revendication 1, dans lequel la concentration de chaque amorce de marquage est de 0,1 à 300 fois, de préférence de 5 à 30 fois inférieure à la concentration de chaque amorce d'amplification.

**3.** Procédé selon les revendications 1 et 2, dans lequel chaque amorce de marquage contient au moins un marqueur choisi dans le groupe constitué par les colorants fluorescents, les marqueurs chimioluminescents, les marqueurs électroluminescents, les marqueurs d'affinité ou de liaison, les marqueurs spécifiques de position et/ou les marqueurs présentant des propriétés physiques spécifiques telles qu'une taille, une masse ou une rotation différente.

**4.** Procédé selon les revendications 1 à 3, dans lequel les amorces de marquage contiennent des colorants fluorescents et dans lequel chaque type d'amorce de marquage est marqué avec un colorant fluorescent différent, de sorte que chaque type d'amorce de marquage se **caractérise par** des propriétés de spectre d'excitation et/ou d'émission, des propriétés de durée de vie, des propriétés de polarisation, des propriétés de transfert d'énergie par résonance de fluorescence (FRET), des rendements quantiques, une stabilité à la lumière ou un nombre de triplets de fluorochromes différents.

**5.** Procédé selon les revendications 1 à 4, dans lequel le marqueur est fixé de manière permanente ou temporaire à l'amorce de marquage, de préférence à l'extrémité 5' de l'amorce de marquage.

**6.** Procédé selon les revendications 1 à 5, dans lequel la réaction d'amplification et le contrôle sont réalisés dans un système homogène.

**7.** Procédé selon les revendications 1 à 6, dans lequel l'agent polymérisant est une enzyme, de préférence une ADN-polymérase, de préférence une ADN-polymérase thermostable, de manière tout particulièrement préférée une ADN-polymérase thermostable qui ne présente pas l'activité d'une 5'$\rightarrow$3' exonucléase.

**8.** Procédé selon les revendications 1 à 7, dans lequel un échantillon de séquences d'acide nucléique cibles est choisi dans un groupe comprenant l'ADN génomique, l'ADNc, l'ADN monocaténaire, l'ADN bicaténaire, l'ADN plasmidique, l'ARN, les mélanges d'ADN avec d'autres molécules, l'ADN ou l'ARN provenant de sources humaines ou d'autres sources telles que des mammifères, des vertébrés, des invertébrés, des bactéries, des virus, des levures, des champignons ou des végétaux.

**9.** Procédé selon les revendications 1 à 8, dans lequel le contrôle des propriétés spécifiques desdits produits de la réaction d'amplification est réalisé par des techniques chromatographiques telles que la CLHP, la chromatographie FPLC, l'électrophorèse capillaire, l'électrophorèse sur gel ou par des techniques électrochimiques ou spectroscopiques de masse ou, de préférence, par des techniques de fluorescence telles que la spectroscopie par polarisation de fluorescence, la spectroscopie de durée de vie de fluorescence, l'analyse de distribution de l'intensité de fluorescence (FIDA), l'analyse par dichroïsme de fluorescence, l'analyse de l'intensité de fluorescence, l'analyse par transfert d'énergie par résonance en fluorescence (FRET), l'analyse spectroscopique du spectre d'excitation et/ou d'émission ou, en particulier, par spectroscopie à corrélation de fluorescence.

**10.** Procédé selon les revendications 1 à 9, dans lequel le contrôle du degré d'allongement d'une amorce de marquage comprend la détection qualitative de nucléotides variants contenus dans un échantillon de séquences d'acide nucléique cibles, dans la mesure où l'amorce de marquage allongée est indicatrice de la présence d'un nucléotide variant et une amorce de marquage non allongée est indicatrice de l'absence d'un nucléotide variant.

**11.** Procédé selon les revendications 1 à 9, dans lequel le contrôle de la quantité d'amorces de marquage allongées comprend la quantification de séquences d'acides nucléiques cibles, en particulier le génotypage d'échantillons de séquences d'acide nucléique cibles, de préférence (i) en calculant le rapport des valeurs de signaux enregistrés individuellement (signal A/ signal B), ou (ii) en soustrayant la valeur du signal B de la valeur du signal A.

**12.** Utilisation du procédé selon les revendications 1 à 11 pour la détection de la présence ou de l'absence d'un ou plusieurs nucléotides variants associés à un état ou à une maladie héréditaire ou acquis.

**13.** Utilisation du procédé selon les revendications 1 à 11 pour le criblage diagnostique d'échantillons de séquences d'acides nucléiques cibles pour des états ou des maladies héréditaires ou acquis et pour le criblage de prédispositions à de tels états et maladies.

**14.** Utilisation du procédé selon les revendications 1 à 11 pour la quantification de séquences d'acides nucléiques cibles, en particulier pour le génotypage de séquences d'acides nucléiques cibles, la détermination de la concentration d'ADN, d'ARN, d'ARNm ou d'ARN viral dans un échantillon, ou la détermination du nombre de copies de séquences d'acides nucléiques cibles, en particulier la détermination du nombre de copies de séquences d'acides nucléiques cibles dans des échantillons provenant de tissu tumoral.

**15.** Trousse pour la détection de la présence ou de l'absence de nucléotides variants contenus dans un échantillon de séquences d'acides nucléiques cibles au moyen d'un procédé selon les revendications 1 à 11, ladite trousse comprenant :

• des nucléosides triphosphates, ou des dérivés fonctionnels de ceux-ci
• un agent polymérisant
• au moins une paire d'amorces d'amplification spécifiques d'une cible capables de s'hybrider sur des séquences d'acides nucléiques cibles
• au moins un jeu d'amorces de marquage, chaque jeu étant constitué d'au moins deux types d'amorces de marquage capables de s'hybrider sur des séquences d'acides nucléiques cibles correspondantes côté 3' par rapport auxdites amorces d'amplification, de sorte que chaque jeu d'amorces de marquage est semi-niché par rapport à la paire correspondante d'amorces d'amplification, et dans lequel les amorces de marquage semi-nichées sont présentes à des concentrations inférieures à celles de la paire d'amorces d'amplification,
les au moins deux types d'amorces de marquage d'un jeu d'amorces de marquage étant **caractérisés en ce qu'**ils ont des séquences de nucléotides essentiellement similaires à l'exception d'au moins le nucléotide 3'-terminal, qui est différent pour chaque type d'amorce de marquage et complémentaire du nucléotide variant, si bien qu'une amorce de marquage allongée est synthétisée si le nucléotide 3'-terminal de l'amorce de marquage est complémentaire du nucléotide correspondant dans la séquence d'acides nucléiques cible, et si bien qu'essentiellement aucun ou un bruit de fond négligeable d'amorce de marquage allongée n'est ou est synthétisée si ledit nucléotide terminal n'est pas complémentaire du nucléotide correspondant dans la séquence d'acide nucléique cible et chaque type d'amorce de marquage étant en outre **caractérisé en ce qu'**il porte un marqueur différent et pouvant être différencié.

**16.** Trousse selon la revendication 15 comprenant au moins un échantillon de séquences d'acide nucléique étalon interne.

# Table 1

| Polymorphism | GenBank® Acc. No. | Amplification Primer (5´- 3´) | | Labeling Primer (5´- 3´) | |
|---|---|---|---|---|---|
| | | Sense | Reverse | Wildtype Allele | Mutant Allele |
| Factor V $G_{1691}A$ | M16967 | AATTGGTTCCAGCG AAAG | AGCCAGGAGACC TAACAT | GGACAAAATACCTGT ATTCCTC | GGACAAAATACCTGTA TTCCTT |
| Prothrombin $G_{20210}A$ | M17262 | GGGATGGGAAATAT GGCT | CCAGGTGGTGGA TTCTTA | CAATAAAAGTGACTC TCAGCG | CAATAAAAGTGACTCT CAGCA |
| MTHFR $C_{677}T$ | NM005957 | CCTGACTGTCATCC CTATTG | AGGACGGTGCGG TGAGAGTG | CTGCGTGATGATGAA ATCGG | CTGCGTGATGATGAAA TCGA |

# Figure 1

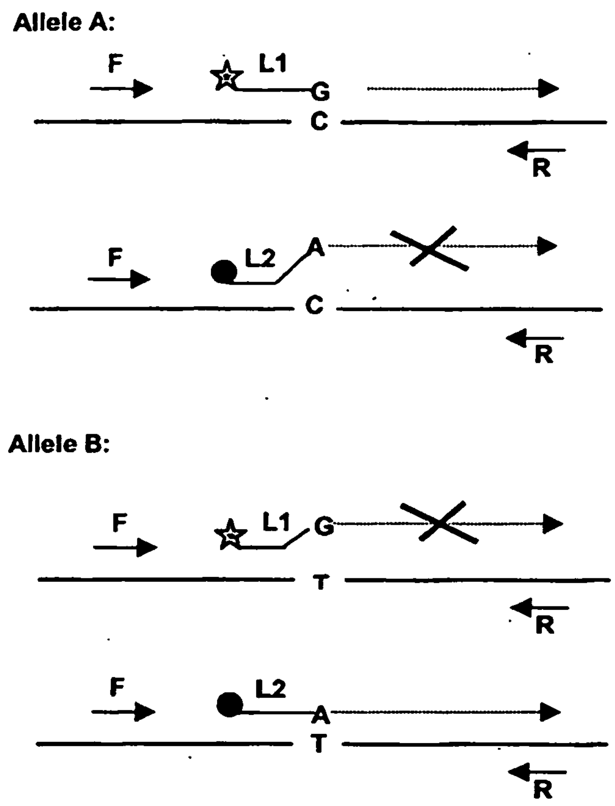
Allele A:
F
L1
G
C
R
F
L2
A
C
R
Allele B:
F
L1
G
T
R
F
L2
A
T
R

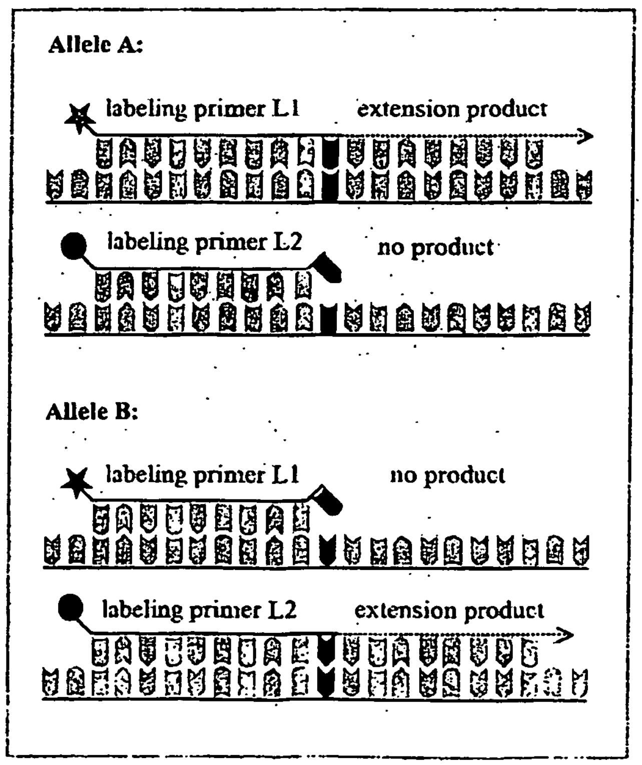
Allele A:
labeling primer L1
extension product
labeling primer L2
no product
Allele B:
labeling primer L1
no product
labeling primer L2
extension product

# Figure 2

A)

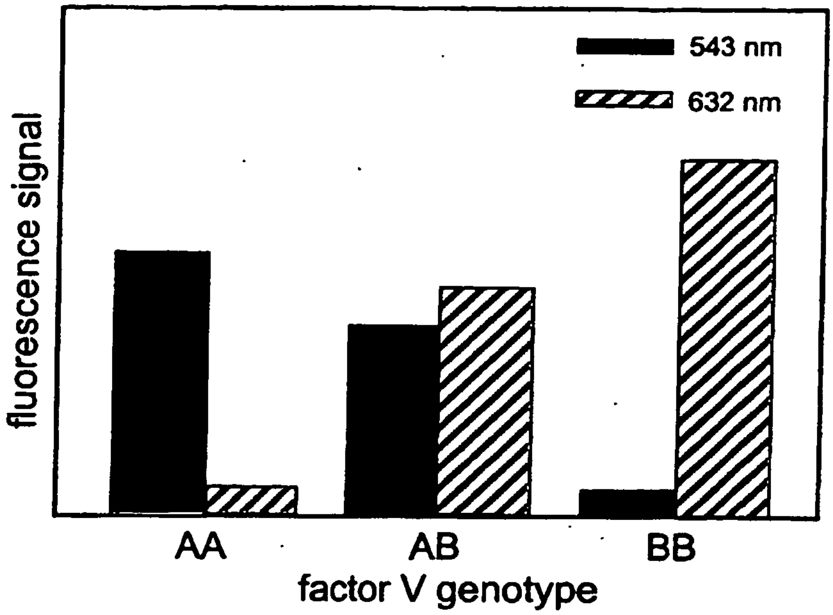


B)

| ratio fluorescence signal 543/633 nm (mean ± SD) | |
|---|---|
| genotype AA | 10.99 ± 2.43 |
| genotype AB | 0.65 ± 0.04 |
| genotype BB | 0.04 ± 0.01 |

# Figure 3

A)

B)

C)

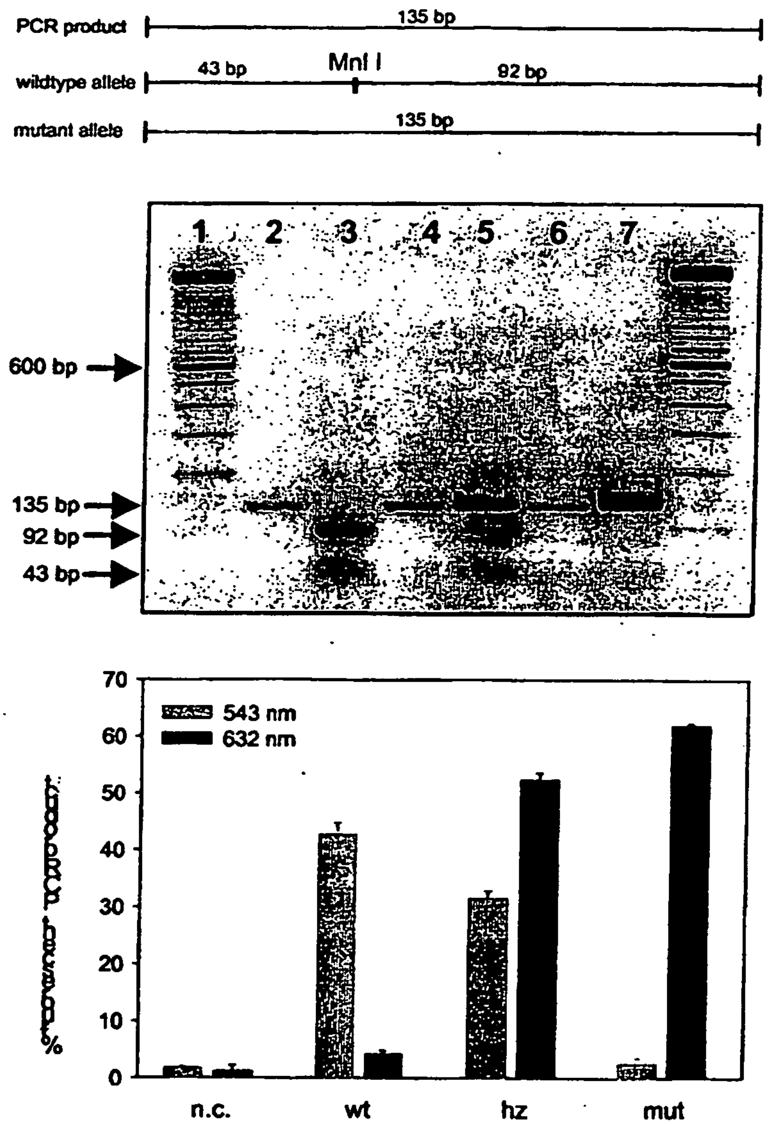


D)

| | ratio 543 / 632 nm ± SD | 95 % confidence interval |
|---|---|---|
| wt | 10.35 ± 1.58 | 9.37 - 11.33 |
| hz | 0.60 ± 0.03 | 0.58 - 0.62 |
| mut | 0.04 ± 0.02 | 0.03 - 0.05 |

# Figure 4

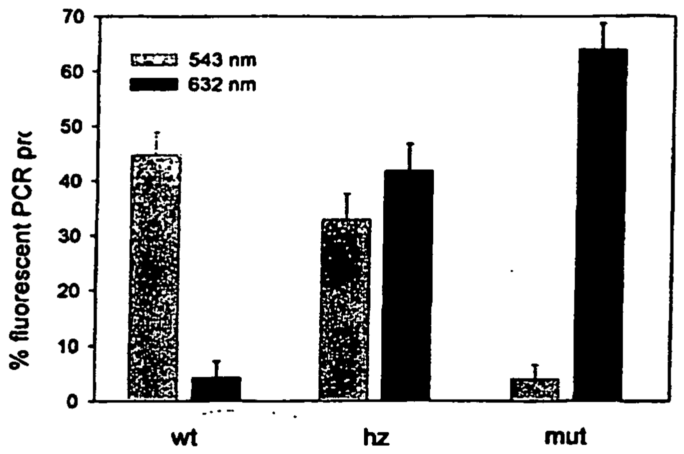


| factor V | ratio 543 / 632 nm ± SD n = 36 | 95 % confidence interval |
|---|---|---|
| wt | 10.99 ± 2.43 | 9.49 - 12.49 |
| hz | 0.65 ± 0.04 | 0.63 - 0.67 |
| mut | 0.04 ± 0.01 | 0.03 - 0.05 |

# Figure 5

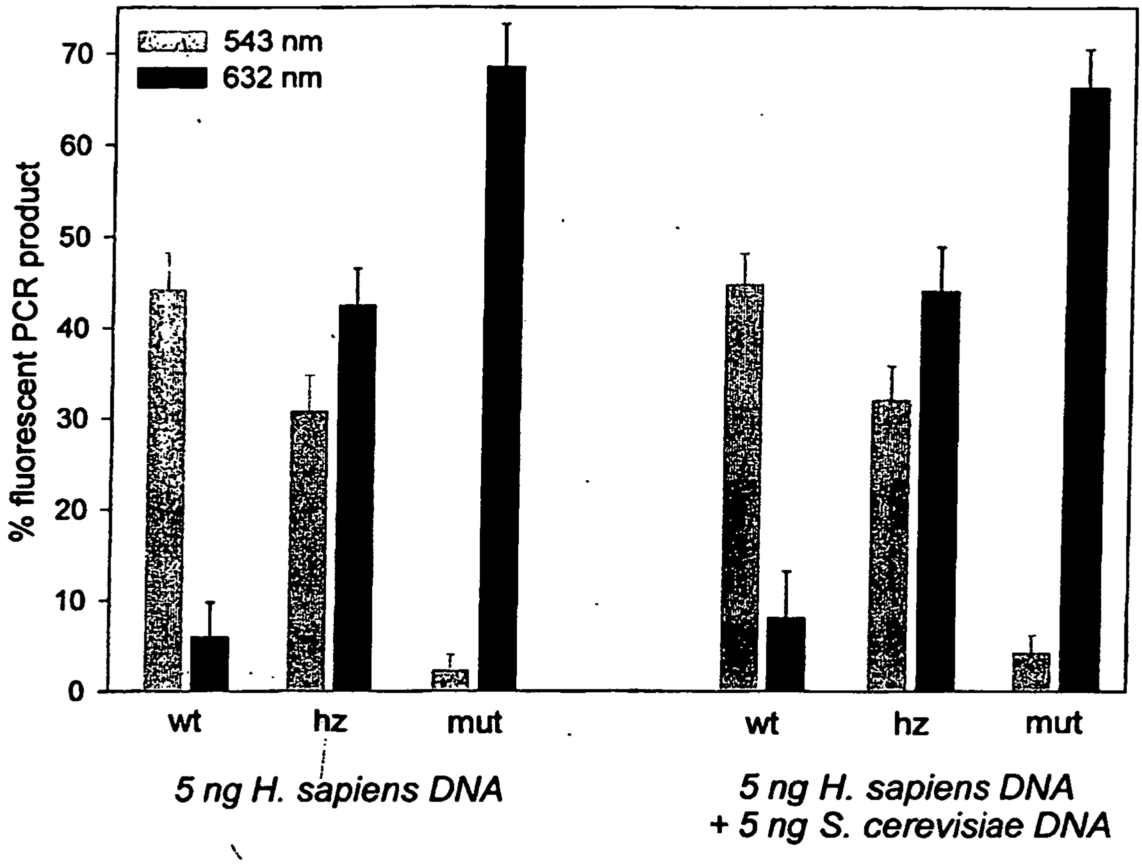
543 nm
632 nm
% fluorescent PCR product
70
60
50
40
30
20
10
0
wt
hz
mut
wt
hz
mut
5 ng H. sapiens DNA
5 ng H. sapiens DNA
+ 5 ng S. cerevisiae DNA

# Figure 6

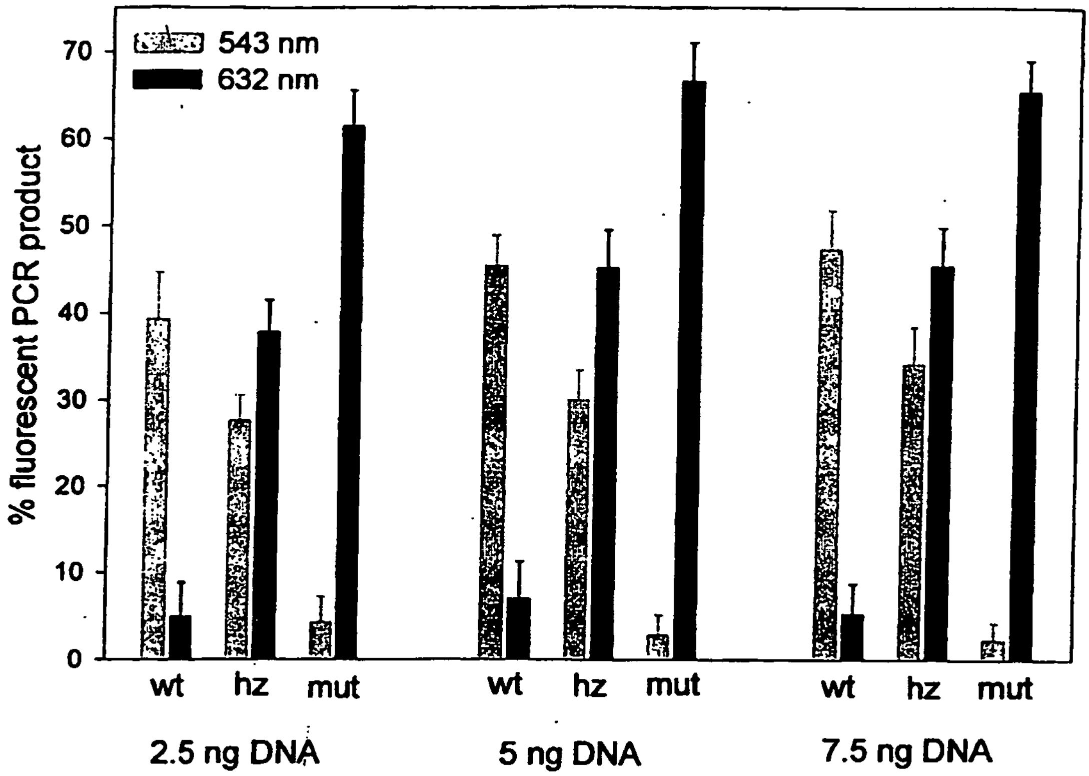

543 nm
632 nm
% fluorescent PCR product
0
10
20
30
40
50
60
70
wt
hz
mut
wt
hz
mut
wt
hz
mut
2.5 ng DNA
5 ng DNA
7.5 ng DNA

# Figure 7

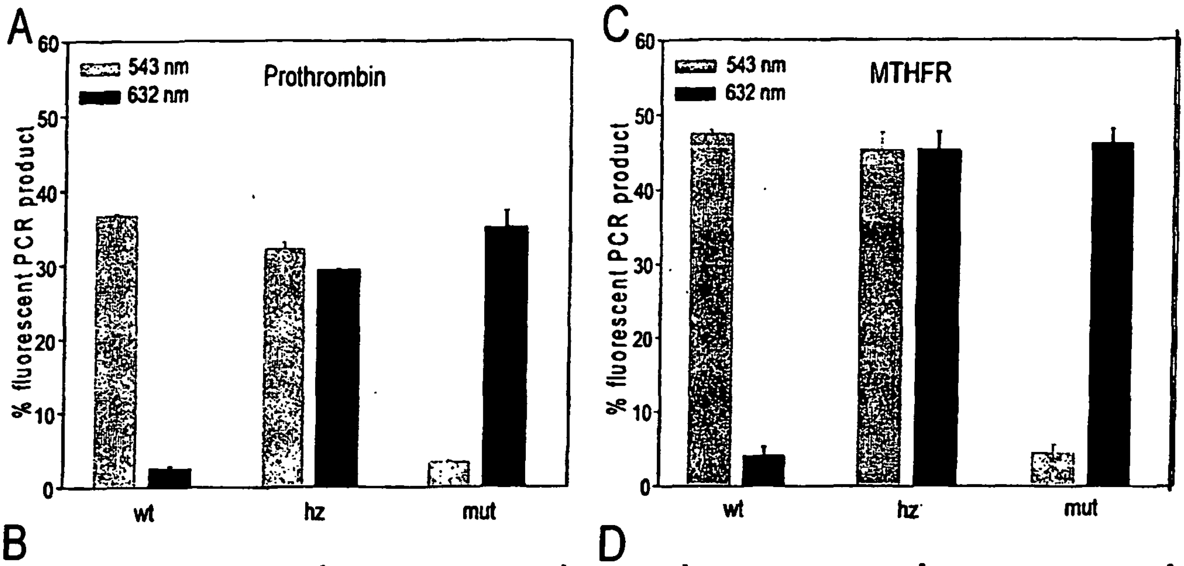


B

| Prothrombin | ratio 543 / 632 nm ± SD; n = 36 | 95 % confidence interval |
|---|---|---|
| wt | 12.17 ± 3.87 | 9.77 - 14.57 |
| hz | 1.06 ± 0.10 | 1.00 - 1.12 |
| mut | 0.12 ± 0.03 | 0.10 - 0.14 |

D

| MTHFR | ratio 543 / 632 nm ± SD; n = 36 | 95 % confidence interval |
|---|---|---|
| wt | 13.23 ± 3.66 | 10.96 - 15.50 |
| hz | 1.00 ± 0.06 | 0.96 - 1.04 |
| mut | 0.11 ± 0.02 | 0.10 - 0.11 |

SEQUENCE LISTING

<110> Evotec Analytical Systems GmbH

<120> Specific Multiplex Analysis of Nucleic Acids

<130> 010701wo

<140> PCT/EP01/03397
<141> 2001-03-26

<150> US60/191760
<151> 2001-03-24

<160> 14

<170> PatentIn Ver. 2.1

<210> 1
<211> 18
<212> DNA
<213> Artificial Sequence

<220>
<223> Description of Artificial Sequence: Amplification-Primer

<400> 1
aattggttcc agcgaaag 18

<210> 2
<211> 18
<212> DNA
<213> Artificial Sequence

<220>
<223> Description of Artificial Sequence: Amplification primer

<400> 2
gggatgggaa atatggct 118

<210> 3
<211> 20

<212> DNA
<213> Artificial Sequence

<220>
<223> Description of Artificial Sequence: Amplification Primer

<400> 3
cctgactgtc atccctattg 120

<210> 4
<211> 18
<212> DNA
<213> Artificial Sequence

<220>
<223> Description of Artificial Sequence: Amplification Primer

<400> 4
agccaggaga cctaacat 118

<210> 5
<211> 18
<212> DNA
<213> Artificial Sequence

<220>
<223> Description of Artificial Sequence:

Amplification-Primer

<400> 5
ccaggtggtg gattctta 118

<210> 6
<211> 20
<212> DNA
<213> Artificial Sequence

<220>
<223> Description of Artificial Sequence:

Amplification-Primer

<400> 6
aggacggtgc ggtgagagtg 120

<210> 7
<211> 22
<212> DNA
<213> Artificial Sequence

<220>
<223> Description of Artificial Sequence:

Labeling-Primer

<400> 7
ggacaaaata cctgtattcc tc 122

<210> 8
<211> 21
<212> DNA
<213> Artificial Sequence

<220>
<223> Description of Artificial Sequence:

Labeling-Primer

<400> 8
caataaaagt gactctcagc g 121

<210> 9
<211> 20
<212> DNA
<213> Artificial Sequence

<220>
<223> Description of Artificial Sequence:

Labeling-Primer

<400> 9
ctgcgtgatg atgaaatcgg 120

<210> 10
<211> 22
<212> DNA
<213> Artificial Sequence

<220>
<223> Description of Artificial Sequence:

Labeling-Primer

<400> 10
ggacaaaata cctgtattcc tt 22

<210> 11
<211> 21
<212> DNA
<213> Artificial Sequence

<220>
<223> Description of Artificial Sequence:

Labeling-Primer

<400> 11
caataaaagt gactctcagc a 21

<210> 12
<211> 20
<212> DNA
<213> Artificial Sequence

<220>

<223> Description of Artificial Sequence:

Labeling-Primer

<400> 12
ctgcgtgatg atgaaatcga 20

<210> 13
<211> 24
<212> DNA
<213> Artificial Sequence

<220>
<223> Description of Artificial Sequence: PCR-Primer

<400> 13
gggctaatag gactacttct aatc 24

<210> 14
<211> 18
<212> DNA
<213> Artificial Sequence

<220>
<223> Description of Artificial Sequence: PCR-Primer

<400> 14
agccaggaga cctaacat 18